# EUROPÄISCHE PATENTSCHRIFT

(11) **EP 0 432 425 B1**
(45) Veröffentlichungstag und Bekanntmachung des Hinweises auf die Patenterteilung: **01.12.1993**
(21) Anmeldenummer: 90120529.4
(22) Anmeldetag: 26.10.1990
(51) Int. Cl.: A61M 5/142

(54) **Vorrichtung zur Infusion**
Infusion device
Dispositif d'infusion

(30) Priorität: 29.11.1989 DE 3939247
(43) Veröffentlichungstag der Anmeldung: 19.06.1991
(73) Patentinhaber: Fresenius AG, 61350 Bad Homburg (DE)
(72) Erfinder: Polaschegg, Hans-Dietrich, W-6370 Oberursel 4 (DE)
(74) Vertreter: Fuchs Mehler Weiss

(56) Entgegenhaltungen:
- EP-A- 0 238 227
- EP-A- 0 293 592
- EP-A- 0 319 279
- EP-A- 0 343 501
- DE-A- 3 329 977

## Beschreibung

Die Erfindung bezieht sich auf eine Vorrichtung zur dosierten, kontinuierlichen und gleichzeitigen Infusion von mehreren Infusionslösungen oder Medikamentenlösungen nach dem Oberbegriff des Hauptanspruchs.

Bei der Infusion von Medikamentenlösungen oder Infusionslösungen werden dem Patienten durch Betätigung der Infusionspumpe in einer vorgegebenen Zeiteinheit bestimmte Volumenmengen zugeführt. Dabei ist es sowohl aus Gründen der Betriebssicherheit als auch aus Gründen der medizinischen Sicherheit des Patienten erforderlich, die zuzuführende Lösungsmenge exakt einzuhalten. Es werden deshalb erhebliche Anforderung an die sicherheitstechnische Ausrüstung einer derartigen Vorrichtung gestellt.

Bei bestimmten Anforderungsbedingungen ist es nötig, die jeweils dem Patienten zuzuführende Volumenmenge exakt einzuhalten, um sowohl eine Vergrößerung als auch eine Verkleinerung der Menge zu verhindern. Derartige Ausgestaltungen erfordern auch einen sehr hohen apparatetechnischen Aufwand. Man ist deshalb bestrebt, die Anforderungen etwas zu senken und die gesamte sicherheitstechnische Ausgestaltung zu vereinfachen. Bei bestimmten Arten von dem Patienten zuzuführenden Lösungen bzw. bei bestimmten medizinischen Indikationen ist es akzeptierbar, wenn dem Patienten eine geringere Lösungsmenge als die vorgegebene Lösungsmenge zugeführt wird. Das sich hieraus ergebende medizinische Risiko wird dabei geringer eingestuft, da durch routinemäßige Kontrollen durch das Bedienungspersonal oder durch andere technische Überwachungsmöglichkeiten eine Minderförderung leichter entdeckt und ausgeglichen werden kann. Es ist somit möglich, die durch die Minderförderung hervorgerufenen Auswirkungen durch gezielte Maßnahmen innerhalb eines größeren Zeitraumes auszugleichen.

Demgegenüber ist es in vielen Anwendungsfällen unter allen Umständen zu vermeiden, dem Patienten in unkontrollierter Weise eine Mehrförderung der zu verabreichenden Lösungen zuzuführen. Derartige Mehrförderungen können sowohl durch apparatetechnische Fehler als auch durch übliche Bedienungsfehler des Personals hervorgerufen werden. Während es bei apparatetechnischen Fehlern unter Verwendung eines Durchflußmessers möglich ist, eine Notabschaltung des Systems vorzunehmen bzw. ein Alarmsignal auszulösen, erfordert die Vermeidung von Bedienungsfehlern zusätzliche konstruktive Maßnahmen.

Ein Gefahrenmoment ergibt sich aus bedienungstechnicher Sicht insbesondere beim Beginn, bei einer gewollten Unterbrechung oder bei einer Beendigung der Infusion. Sofern bei diesen Betriebszuständen ein freier Durchlaß der entsprechen Schläuche oder Katheder vorhanden ist, kann durch den Einfluß der Schwerkraft eine unkontrollierte Menge an Infusionslösung in den Körper des Patienten strömen. Diese Gefahr besteht beispielsweise dann, wenn zu Beginn der Infusion eine Infusionsleitung in die Infusionspumpe eingelegt wird. Ebenso besteht die Gefahr, wenn die Infusion unterbrochen oder abgebrochen wird und wenn bei dieser Gelegenheit die Infusionsleitung aus der Pumpe entnommen werden soll.

Das DE-U-79 13 478 zeigt eine Infusionspumpe, bei welcher im Bereich der an dem Gehäuse gelagerten Tür ein mechanisches Sicherheitssystem vorgesehen ist, welches verhindert, daß die Tür geschlossen wird, solange der Schlauch nicht vollständig und richtig eingelegt ist. Dieses mechanische System verhindert lediglich das Schließen der Tür, es ist jedoch nicht in der Lage, ein unbeabsichtigtes Öffnen der Tür festzustellen. Im letzteren Falle besteht während des Betriebs der Pumpe die Möglichkeit, daß sich der Schlauch löst und herausrutscht.

Auch die EP-A-0238227 zeigt eine Infusionseinrichtung, bei der sich eine peristaltische Pumpe in einem Pumpenraum befindet, der zum Einlesen und Entnehmen des Schlauchs mittels einer Tür zugänglich gemacht ist. Ein Mechanismus schließt beim Öffnen der Tür ein Ventil im Pumpenraum und Öffnet dieses beim Schließen der Tür. Dieser einfache Türöffnungsmechanismus stellt eine Sicherheitseinrichtung dar, die allerdings für ein Multifunktionssystem nicht geignet ist.

Die DE-A-33 29 977-sie liegt dem Oberbegriff des Anspruchs 1 zugrunde-zeigt eine Mehrfach-Infusionsanordnung, bei welcher jeweils ein Ventil einem Vorratsbehälter nachgeordnet ist. Stromab des Ventils führt eine Leitung zu einer gemeinsamen Infusionspumpe, welche sämtliche Fluidströme von den Vorratsbehältern zu den Patienten fördert. Daraus ergibt sich, daß es zwar möglich ist, mittels der einzelnen Ventile die Zuleitung zwischen dem Vorratsbehälter und der gemeinsamen Infusionspumpe zu verschließen, bei einem unbeabsichtigten Öffnen der Pumpe erfolgt jedoch ein freier Druchfluß zu dem Patienten, ohne daß die Möglichkeit besteht, Sperrmechanismen vorzusehen.

Der Erfindung liegt die Aufgabe zugrunde, eine Vorrichtung der eingangs genannten Art zu schaffen, welche bei einfachem Aufbau und betriebssicherer Bedienbarkeit eine ungewollte Zuführung einer oder mehrerer Infusionslösungen zum Patienten ausschließt.

Die Aufgabe wird gelöst durch die Merkmalskombination des Hauptanspruchs, die Unteransprüche zeigen vorteilhafte Ausgestaltungen der Erfindung.

Die erfindungsgemäße Vorrichtung zeichnet sich durch eine Reihe erheblicher Vorteile aus. Während bei einer eingelegten Infusionsleitung der Pumpmechanismus der Infusionspumpe den freien Durchfluß durch die Infusionsleitung sperrt und somit als Ventil wirkt, ist die Infusionsleitung bei Entnahme aus der Pumpe durch den Pumpmechanismus nicht mehr gesperrt oder unterbrochen. Üblicherweise wird deshalb eine manuell zu betätigende Klemme vorgesehen, mit der die Infusionsleitung geschlossen werden kann. Beim Einlegen der Infusionsleitung in den Pumpmechanismus kann diese manuelle Klemme geöffnet werden, ohne daß die Gefahr einer Mehrförderung besteht, da der Pumpmechanismus selbst als Sperrventil wirkt. Wenn nun durch das Bedienungspersonal zu einem beliebigen Zeitpunkt die Infusionsleitung aus der Infusionspumpe genommen wird, so ist es nach dem Stand der Technik erforderlich, zunächst die manuelle Klemme zu schließen und danach die Leitung entnehmen. Wird die manuelle Klemme nicht oder nicht vollständig geschlossen, so strömt die im Infusionsbehälter befindliche Infusionslösung in unkontrollierter Weise durch die Infusionsleitung und gelangt in den Körper des Patienten.

Die erfindungsgemäße Vorrichtung weist somit den Vorteil auf, daß die Freigabe der Infusionsleitung oder Förderleitung unabhängig von der Betätigung der manuellen Klemme erfolgen kann, so daß die Betriebssicherheit der Vorrichtung nicht durch Handhabungen des Bedienungspersonals beeinflußt werden kann.

Die aus dem Stand der Technik bekannten Vorrichtungen verfügen zwar über einen Mechanismus, welcher erst nach zwei Betätigungsschritten den Querschnitt der Infusionsleitung oder Durchfußleitung freigibt. Üblicherweise wird zwischen den beiden Betätigungsschritten ein Warnsignal abgegeben, welches das Bedienungspersonal auf die mögliche Gefahr aufmerksam machen soll. Eine zwangsweise Verschließung der Infusionsleitung erfolgt jedoch nicht, so daß Bedienungsfehler nicht vollständig auszuschließen sind. Es ist dabei insbesondere darauf hinzuweisen, daß vielfach derartige Bedienungsvorgänge durch angelerntes und weniger sachkundiges Personal durchgeführt werden und daß weiterhin vielfach, insbesondere in Notfällen, keine ausreichende Zeit zur Verfügung steht, um die einzelnen Bedienungsschritte nochmals nachkontrollieren zu können. Deshalb besteht bei den aus dem Stand der Technik bekannten Vorrichtungen ein Sicherheitsrisiko.

Die erfindungsgemäße Vorrichtung ist insbesondere bei Multi-Infusionssystemen besonders vorteilhaft, da eine manuelle Betätigung von Klemmen bzw. eine entsprechende Überwachung der einzelnen Handhabungsschritte durch Kombination mehrerer Infusionspumpen erheblich erschwert wird und einen großen apparatetechnischen Aufwand darstellt. Erfindungsgemäß ist es möglich, mittels eines relativ einfachen Sicherheitsmechanismus das in der Förderleitung bereits vorgesehene Ventil zu betätigen bzw. ein einfach aufgebautes zusätzliches Ventil vorzusehen. Die gesamte Vorrichtung verfügt somit über ein Höchstmaß an Betriebssicherheit und ist auch durch Fehlbedienungen nicht negativ beeinflußbar, da der Türdetektor der peristaltischen Pumpe ein Öffnen bzw. Schließen der Tür selbstätig feststellt. Als besonders vorteilhaft erweist es sich dabei, daß keine zusätzlichen, störungsanfälligen Sicherheitseinrichtungen erforderlich sind und daß beispielsweise keine weiteren Durchflußsensoren oder ähnliches vorgesehen sein müssen, welche den Herstellungsaufwand der gesamten Anlage verteuern.

In einer bevorzugten Weiterbildung der Erfindung ist vorgesehen, daß das Ventil elektrisch betätigbar ist. Alternativ dazu ist es auch möglich, das Ventil mechanisch zu betätigen. Vorteile ergeben sich für beide Ausgestaltungsformen, bei der mechanischen Betätigung ist es möglich, das Ventil und/oder den Betätigungsmechanismus direkt mit der Tür der Pumpe zu koppeln, während eine elektrische Betätigung die Möglichkeit schafft, das Ventil in einer größeren Entfernung zur Pumpe anzuordnen.

Bei einer mechanischen Betätigung kann das Ventil in die Pumpe integriert sein, wobei dann das Ventil mittels der Tür direkt betätigbar ist.

Bei einer mechanischen Betätigung des Ventils ist es besonders günstig, dieses in Form eines Federelements auszubilden, welches bei geöffneter Tür, d.h. ohne Kraftbeaufschlagung die Förderleitung oder die Infusionsleitung abklemmt. Bei einem Schließen der Tür ist das Federelement beispielsweise spreizbar, so daß der Schlauch freigegeben wird, so daß die Durchströmung nicht beeinträchtigt wird.

Als besonders günstig erweist es sich dabei, wenn das Federelement auf der der Tür abgewandten Seite an einem Widerlager anliegt, so daß keine zusätzlichen Hebemechanismen oder ähnliches erforderlich sind, sondern die Tür selbst als Betätigungshebel für das Federelement wirkt.

Bei einer elektrischen Betätigung des Ventils ist es besonders günstig, wenn sich dieses im stromlosen Zustand in Schließstellung befindet, da auch bei einem plötzlichen Spannungsabfall oder einer Unterbrechung der Energieversorgung ein unkontrolliertes Einströmen von Infusionslösung in die Patientenleitung verhindert wird. Um das Ventil zu betätigen, kann der Türdetektor beispielsweise über die Steuereinheit mit dem Ventil elektrisch verbunden sein, so daß ein geeigneter Impuls abgegeben wird, um das Ventil zu öffnen bzw. zu schließen.

Die erfindungsgemäße Vorrichtung gestattet unterschiedliche Ausgestaltungsformen des Ventils. So ist es möglich, das Ventil einstückig mit dem in der Förderleitung angeordneten Ventil auszubilden, d.h. das Ventil der Förderleitung sowohl als Sicherheitsventil in Verbindung mit dem Durchflußsensor als auch als Sicherheitsventil beim Öffnen der Tür der Pumpe zu verwenden. Alternativ dazu ist es jedoch auch möglich, das Ventil als separates Ventil auszugestalten, um es beispielsweise an anderer Stelle der Leitungsanordnung der Förderleitungen einzubauen.

Im folgenden wird die Erfindung anhand eines Ausführungsbeispiels in Verbindung mit der Zeichnung beschrieben. Dabei zeigt:
- Fig. 1: eine schematische Darstellung einer Mehrfach-Infusionseinrichtung,
- Fig. 2: eine schematische Darstellung einer erfindungsgemäßen Ventilausgestaltung im geöffneten Zustand und
- Fig. 3: eine schematische Darstellung der in Fig. 2 gezeigten Ventilausgestaltung im geschlossenen Zustand.

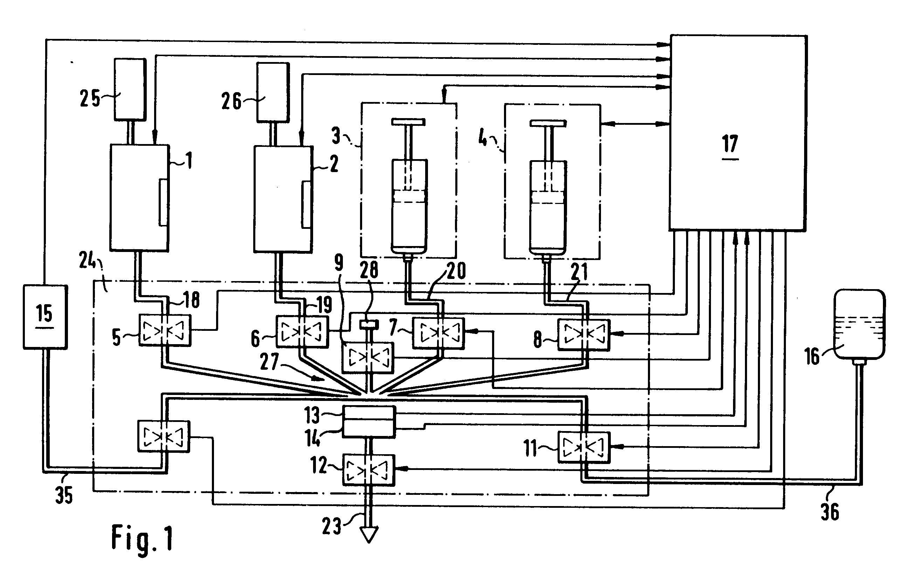

Die in Fig. 1 dargestellte Mehrfach-Infusionseinrichtung umfaßt zwei linear-peristaltische Infusionspumpen 1 und 2, zwei Spritzen-Infusionspumpen 3 und 4, eine Schwerkraft-Infusionseinrichtung 16 und eine CVD-Meßeinrichtung 15. Die Infusionspumpen 1 und 2 sind jeweils an Infusionsbehälter 25 und 26 angeschlossen. Jede der Infusionspumpen 1 und 2 ist, ebenso wie die Schwerkraft-Infusionseinrichtung 16 an eine Förderleitung angeschlossen, welche die Bezugszeichen 18, 19, 20, 21 und 36 aufweisen. In jeder dieser Förderleitungen befindet sich ein Klemmventil 5, 6, 7, 8, und 11, wobei die Ventile signalmäßig mit einer Rechen- und Steuereinheit 17 verbunden sind. Diese ist gleichzeitig an die Pumpen 1, 2, 3 und 4 angeschlossen und steuert diese nach einem vorbestimmten Programm. Die Klemmventile 5, 6, 7, 8 und 11 sowie die Zuleitung 35 zur CVD-Meßeinrichtung 15 und die Förderleitung 36 der Schwerkraft-Infusionseinrichtung sind über ein Übergangsstück 27 gemeinsam an eine Patientenleitung 23 angeschlossen. In dieser ist ein Luftsensor 13 sowie ein Durchflußsensor 14 vorgesehen. Stromab des Durchflußsensors ist ein weiteres Ventil 12 angeordnet. Der Luftsensor 13, der Durchflußsensor 14 und das Ventil 12 sind ebenfalls an die Rechen- und Steuereinheit 17 angeschlossen.

An dem Übergangsstück 27 ist zusätzlich noch eine Belüftungseinrichtung 28 angebracht, welche über ein Ventil 9, welches ebenfalls mit der Rechen- und Steuereinheit 17 verbunden ist, betätigbar ist. Es kann auch ein hydrophobes Filter und ein nicht gezeigtes Rückschlagventil für die Belüftungseinrichtung vorgesehen sein.

Die unterschiedlichen Infusionseinrichtungen können somit mit Hilfe der Rechen -und Steuereinheit 17 betätigt werden, wobei die jeweiligen Ventile die Möglichkeit bieten, gezielte Mengen an Infusionslösung zuzuführen. Das Sicherheitsventil 12 kann geschlossen werden, wenn die Förderleistung nicht den gewünschten Werten entspricht.

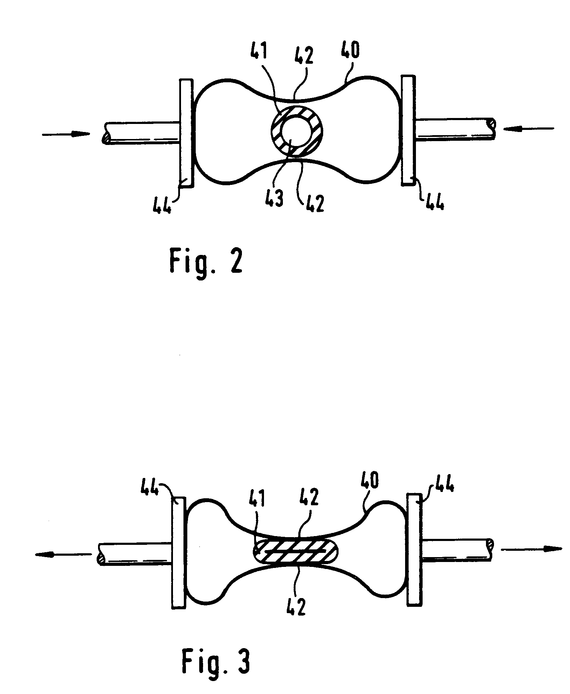

Die beiden peristaltischen Pumpen 1 und 2 sind in üblicher Weise aufgebaut und umfassen einen Rotor mit Druckrollen, welcher in einen Schlauchbogen eingelegt ist. Die Pumpe ist dabei mit einer Tür versehen, um den Pumpenraum zum Einlegen bzw. Entnehmen des Schlauches zugänglich zu machen. Im Bereich der Tür der Pumpe 1, 2 ist eine in den Fig. 2 und 3 schematisch dargestellte Sicherheitseinrichtung angebracht, welche ein Ventil bildet.

Bei dem in Fig. 2 und 3 gezeigten Ausführungsbeispiel ist ein Federelement 40 vorgesehen, welches einen Schlauch 41 umgibt. Das Federelement 40 ist im wesentlichen ringförmig ausgebildet und weist an zwei gegenüberliegenden Seiten nach innen vorspringende Nasen 42 auf. In der nicht belasteten Ausgangsstellung des Federelements 40, welche in Fig. 3 gezeigt ist, drücken die beiden Nasen 42 gegeneinander und quetschen den Schlauch 41, so daß dessen Lumen 43 gedrückt und somit verschlossen wird.

Angrenzend an das Federelement 40 sind Stempel 44 vorgesehen, welche mit einem im einzelnen nicht beschriebenen Mechanismus, welcher mechanischer oder elektrischer Art sein kann, verbunden sind und aufeinander zu bzw. voneinander weg bewegt werden können.

Mittels der Druckstempel 44 kann das Federelement 40, wie in Fig. 2 gezeigt, seitlich gedrückt werden, so daß sich die beiden Nasen 42 voneinander entfernen und den Schlauch 41 freigeben.

Die Erfindung ist nicht auf das gezeigte Ausführungsbeispiel beschränkt, vielmehr ergeben sich für den Fachmann im Rahmen der Erfindung vielfältige Abwandlungs- und Modifikationsmöglichkeiten.

## Patentansprüche

1. Vorrichtung zur dosierten, kontinuierlichen gleichzeitigen Infusion von mehreren Infusionslösungen oder Medikamentenlösungen mit mindestens einer Pumpe (1, 2) mit Förderleitungen (18 - 21), die von den die Infusionslösungen bzw. Medikamentenlösungen aufweisenden Behältern abgehen und über ein Übergangsstück mit einer gemeinsamen Patientenleitung (23) verbunden sind, mit Ventilen (5 - 12), die in jeder Förderleitung vorgesehen sind, mit einem den Durchfluß detektierenden Durchflußsensor (14) in der Patientenleitung (23) und mit einer Steuereinheit, die mit den Ventilen (5 - 12), der Pumpe (1, 2) und dem Durchflußsensor (14) betriebsverbunden sind, wobei der Durchflußsensor (14) stromab der Pumpe angeordnet ist, und die Steuereinheit bei Erkennen eines Fluidstops durch den Durchflußsensor (14) die Schließung der Ventile (5 - 12) veranlaßt,
dadurch gekennzeichnet, daß die Pumpe (1,2) mit jeweils einem Gehäuse u. einer Tür versehen ist, daß das in der Förderleitung einer peristaltischen Pumpe (1, 2) vorgesehene Ventil (5, 6) mit einem Türdetektor der zugehörigen Pumpe (1, 2) derart betriebsverbunden ist, daß beim Öffnen der Tür das Ventil (5, 6) geschlossen ist und beim Schließen der Tür das Ventil (5, 6) geöffnet ist.

2. Vorrichtung nach Anspruch 1, dadurch gekennzeichnet, daß das Ventil (5, 6) elektrisch betätigbar ist.

3. Vorrichtung nach Anspruch 1, dadurch gekennzeichnet, daß das Ventil (5, 6) mechanisch betätigbar ist.

4. Vorrichtung nach Anspruch 3, dadurch gekennzeichnet, daß das Ventil in der Pumpe integriert ist und mittels der Tür betätigbar ist.

5. Vorrichtung nach Anspruch 3 oder 4, dadurch gekennzeichnet, daß (5, 6) das Ventil aus einem Federelement (40) besteht, das bei geöffneter Tür die Förderleitung abklemmt und das beim Schließen der Tür zur Freigabe der Förderleitung spreizbar ist.

6. Vorrichtung nach Anspruch 5, dadurch gekennzeichnet, daß das Federelement (40) auf der der Tür abgewandten Seite an einem Widerlager anliegt.

7. Vorrichtung anch Anspruch 1 oder 2, dadurch gekennzeichnet, daß das Ventil (5, 6) ein elektrisch schaltbares Ventil ist, das sich im stromlosen Zustand in Schließstellung befindet.

8. Vorrichtung nach Anspruch 7, dadurch gekennzeichnet, daß der Türdetektor über die Steuereinheit mit dem Ventil (5, 6) verbunden ist.

9. Vorrichtung nach einem der Ansprüche 1 bis 8, dadurch gekennzeichnet, daß das Ventil (5, 6) einstückig mit dem in der Förderleitung angeordneten Ventil ausgebildet ist.

10. Vorrichtung nach einem der Ansprüche 1 bis 8, dadurch gekennzeichnet, daß das Ventil (5, 6) als separates Ventil ausgestaltet ist.

## Claims

1. An apparatus for the dosed, continuous and simultaneous infusion of a plurality of infusion solutions or medicament solutions, comprising at least one pump (1, 2), conveying conduits (18-21) which pass from containers containing said infusion solutions or medicament solutions and are connected via a transition member to a common patient's conduit (23), valves (5-12) provided in each of said conveying conduits, a flow sensor (14) provided in said patient's conduit (23) for sensing the flow rate, and a control unit operatively connected to said valves (5-12), said pump (1, 2) and said flow sensor (14), said flow sensor (14) being arranged downstream of said pump, and said control unit initiating the closure of said valves (5-12) upon detection of a fluid stop by said flow sensor (14),
characterized in that said pump (1, 2) is respectively provided with a housing and a door and that said valve (5, 6) provided in said conveying conduit of a peristaltic pump (1, 2) is operatively connected to a door detector of said associated pump (1, 2) in such a way that said valve (5, 6) is closed when said door is opened, and opened when said door is closed.

2. An apparatus according to claim 1, characterized in that said valve (5, 6) is electrically actuable.

3. An apparatus according to claim 1, characterized in that said valve (5, 6) is mechanically actuable.

4. An apparatus according to claim 3, characterized in that said valve is integrated into said pump and actuable by means of said door.

5. An apparatus according to claim 3 or 4, characterized in that said valve (5, 6) consists of a spring element (40) which clamps off said conveying conduit in the opened state of said door and can be expanded for releasing said conveying conduit when said door is closed.

6. An apparatus according claim 5, characterized in that said spring element (40) rests on an abutment at the side facing away from said door.

7. An apparatus according to claim 1 or 2, characterized in that said valve (5, 6) is an electrically switchable valve which is in the closing position in the currentless state.

8. An apparatus according to claim 7, characterized in that said door detector is connected to said valve (5, 6) via said control unit.

9. An apparatus according to any of claims 1 through 8, characterized in that said valve (5, 6) is integrally formed with said valve arranged in said conveying conduit.

10. An apparatus according to any of claims 1 through 8, characterized in that said valve (5, 6) is formed as a separate valve.

## Revendications

1. Dispositif pour la perfusion dosée, continue et simultanée de plusieurs solutions de perfusion ou plusieurs solutions de médicaments, comprenant au moins une pompe (1, 2) munie des conduites de refoulement (18 - 21), qui partent des réservoirs contenant les solutions de perfusion ou les solutions de médicaments, et qui sont reliées à une conduite commune (23) aboutissant au patient par l'intermédiaire d'un élément de raccordement, des vannes (5 à 12), qui sont prévues dans les différentes conduites de refoulement, un détecteur d'écoulement (14) qui détecte l'écoulement dans la conduite (23) menant au patient, et une unité de commande qui est connectée fonctionnellement aux vannes (5 à 12), à la pompe (1, 2) et au détecteur d'écoulement (14) en aval de la pompe, et, lorsqu'elle décèle un arrêt d'écoulement du fluide à travers le détecteur d'écoulement (14), l'unité de commande provoque la fermeture des vannes (5 à 12), caractérisé en ce que la pompe (1, 2) est munie d'un boîtier et d'une porte, en ce que la vanne (5, 6) prévue dans la conduite d'une pompe de refoulement péristaltique (1, 2) est reliée fonctionnellement à un détecteur de porte de la pompe (1, 2) de telle manière que la vanne (5, 6) se ferme lorsque la porte s'ouvre et que la vanne (5, 6) s'ouvre lorsque la porte se ferme.

2. Dispositif selon la revendication 1, caractérisé en ce que la vanne (5, 6) peut être commandée électriquement.

3. Dispositif selon la revendication 1, caractérisé en ce que la vanne (5, 6) peut être commandée mécaniquement.

4. Dispositif selon la revendication 3, caractérisé en ce que la vanne est intégrée dans la pompe et peut être actionnée au moyen de la porte.

5. Dispositif selon la revendication 3 ou 4, caractérisé en ce que la vanne (5, 6) est composée d'un élément élastique (40) qui ferme la conduite de refoulement lorsque la porte est ouverte, qui peut s'écarter pour libérer la conduite de refoulement lors de la fermeture de la porte.

6. Dispositif selon la revendication 5, caractérisé en ce que l'élément élastique (40) prend appui sur une butée sur le côté qui est à l'opposé de la porte.

7. Dispositif selon la revendication 1 ou 2, caractérisé en ce que la vanne (5, 6) est une vanne à commande électrique qui est en position de fermeture dans l'état sans courant.

8. Dispositif selon la revendication 7, caractérisé en ce que le détecteur de porte est connecté à la vanne (5, 6) par l'intermédiaire de l'unité de commande.

9. Dispositif selon une des revendications 1 à 8, caractérisé en ce que la vanne (5, 6) est réalisée en une seule pièce avec la vanne agencée dans la conduite de refoulement.

10. Dispositif selon une des revendications 1 à 8, caractérisé en ce que la vanne (5, 6) est constituée par une vanne séparée.
